# EUROPEAN PATENT APPLICATION

(11) **EP 2 168 517 A1**
(43) Date of publication of application: **31.03.2010**
(21) Application number: 09015215.8
(22) Date of filing: 02.08.2007
(51) Int. Cl.: A61B 18/14

(54) **Hanheld electrosurgical instruments having disable handswitches**

(30) Priority: 04.08.2006 US 499590
(62) Divisional of application: 07015191.5
(71) Applicant: Covidien AG, 8200 Schaffhausen (CH)
(72) Inventor: Artale, Ryan, Boulder, CO 80305 (US); Hushka, Dylan, Denver, CO 80224 (US)
(74) Representative: HOFFMANN EITLE

(57) **Abstract**

The present disclosure provides for an electrosurgical forceps for sealing tissue. The forceps comprises at least one handle having at least one shaft member attached thereto. The at least one shaft member having an end effector attached at a distal end thereof. The end effector includes a pair of jaw members being movable from a first position in spaced relation relative to one another to at least one subsequent position wherein the jaw members cooperate to grasp tissue therebetween. Each of the jaw members includes an electrically conductive sealing plate for communicating electrosurgical energy through tissue held therebetween to effect a tissue seal, the electrically conductive sealing plates adapted to connect to an electrosurgical generator. The forceps also include a handswitch operatively coupled to at least one of the at least one handle and the at least one shaft member. The handswitch is adapted to connect to the electrosurgical generator and is selectively actuatable to initiate electrosurgical activation of the forceps. The forceps further include a lockout switch operatively coupled to at least one of the at least one handle and the at least one shaft member. The lockout switch is movable from a first configuration wherein the lockout switch allows actuation of the handswitch to a second configuration wherein the lockout switch prevents actuation of the handswitch and activation of the forceps.

## Description

### BACKGROUND

### Technical Field

The present disclosure relates to a system and method for disabling handswitches of handheld electrosurgical instruments. More particularly, the present disclosure relates to electrical and mechanical arrangements for disabling handswitches which are typically configured to allow the selective application of electrosurgical energy to handheld instruments.

### Background of Related Art

Energy-based tissue treatment is well known in the art. Various types of energy (e.g., electrical, ultrasonic, microwave, cryo, heat, laser, etc.) may be applied to tissue to achieve a desired surgical result. Electrosurgery typically involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the user and applied to the tissue to be treated. A patient return electrode is placed remotely from the active electrode to carry the current back to the generator.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes. When the electrodes are sufficiently separated from one another, the electrical circuit is open and thus inadvertent contact with body tissue with either of the separated electrodes does not cause current to flow.

Various types of instruments are utilized to perform electrosurgical procedures such as monopolar cutting instruments, bipolar electrosurgical forceps, etc. which are further adapted for either endoscopic or open use. Many of these instruments include multiple switching arrangements (e.g., handswitches, foot switches, etc.) which actuate the flow of electrosurgical energy to the instrument. During surgery the user actuates the switching arrangement once the instrument is positioned at a desired tissue site. For this purpose, the handswitches usually include large easily accessible buttons which facilitate selective actuation. There is a need for a system and method which would disable handswitches of handheld electrosurgical instruments to prevent the inadvertent supply of electrosurgical energy.

### SUMMARY

The present disclosure relates to a system and method for disabling handswitches of handheld electrosurgical instruments. In particular, the disclosure provides for mechanical, electrical and electro-mechanical configurations which disable handswitches.

According to one aspect of the present disclosure an electrosurgical forceps for sealing tissue is disclosed. The forceps comprises at least one handle having at least one shaft member attached thereto. The at least one shaft member having an end effector attached at a distal end thereof. The end effector includes a pair of jaw members being movable from a first position in spaced relation relative to one another to at least one subsequent position wherein the jaw members cooperate to grasp tissue therebetween. Each of the jaw members includes an electrically conductive sealing plate for communicating electrosurgical energy through tissue held therebetween to effect a tissue seal, the electrically conductive sealing plates adapted to connect to an electrosurgical generator. The forceps also include a handswitch operatively coupled to at least one of the at least one handle and the at least one shaft member. The handswitch is adapted to connect to the electrosurgical generator and is selectively actuatable to initiate electrosurgical activation of the forceps. The forceps further include a lockout switch operatively coupled to at least one of the at least one handle and the at least one shaft member. The lockout switch is movable from a first configuration wherein the lockout switch allows actuation of the handswitch to a second configuration wherein the lockout switch prevents actuation of the handswitch and activation of the forceps.

The present disclosure also relates to another embodiment of an electrosurgical forceps for sealing tissue.. The forceps comprises at least one handle having at least one shaft member attached thereto. The at least one shaft member having an end effector attached at a distal end thereof. The end effector includes a pair of jaw members being movable from a first position in spaced relation relative to one another to at least one subsequent position wherein the jaw members cooperate to grasp tissue therebetween. Each of the jaw members includes an electrically conductive sealing plate for communicating electrosurgical energy through tissue held therebetween to effect a tissue seal, the electrically conductive sealing plates adapted to connect to an electrosurgical generator. The forceps also include a handswitch operatively coupled to at least one of the at least one handle and the at least one shaft member. The handswitch is adapted to connect to the electrosurgical generator and is selectively actuatable to initiate electrosurgical activation of the forceps. The forceps further include a lockout switch operatively coupled to at least one of said at least one handle and said at least one shaft member. The lockout switch being configured in electrical communication with said handswitch such that both said lockout switch and said handswitch must be electrically closed to allow activation of said forceps.

According to another aspect of the present disclosure another embodiment of an electrosurgical forceps for sealing tissue is disclosed. The forceps comprises at least one handle having at least one shaft member attached thereto. The at least one shaft member having an end effector attached at a distal end thereof. The end effector includes a pair of jaw members being movable from a first position in spaced relation relative to one another to at least one subsequent position wherein the jaw members cooperate to grasp tissue therebetween. Each of the jaw members includes an electrically conductive sealing plate for communicating electrosurgical energy through tissue held therebetween to effect a tissue seal, the electrically conductive sealing plates adapted to connect to an electrosurgical generator. The forceps also include a handswitch operatively coupled to at least one of the at least one handle and the at least one shaft member. The handswitch is adapted to connect to the electrosurgical generator and is selectively actuatable to initiate electrosurgical activation of the forceps. The forceps further include a lockout switch operatively coupled to at least one ratchet interface. The lockout switch includes a first configuration wherein said ratchets are disposed in spaced, non-operative engagement with one another which prevents actuation of said handswitch and a second configuration wherein said ratchets are operatively engaged with one another which allows actuation of said handswitch and activation of said forceps.

### BRIEF DESCRIPTION OF THE DRAWINGS

Various embodiments of the present disclosure are described herein with reference to the drawings wherein:
Fig. 1 is a schematic block diagram of an electrosurgical system according to the present disclosure;
Fig. 2 is a schematic block diagram of a generator according to one embodiment of the present disclosure;
Fig. 3A is a top, perspective view of an open electrosurgical forceps according to one embodiment of the present disclosure;
Fig. 3B is a right, rear perspective view of the forceps of Fig. 3A;
Fig. 3C is an enlarged view of the area of detail of Fig. 3B;
Fig. 3D is a rear view of the forceps shown in Fig. 3A;
Fig. 3E is a perspective view of the forceps of Fig. 3A with parts separated;
Fig. 4 is an internal, side view of the forceps showing the rack and pinion actuating mechanism and the internally disposed electrical connections;
Fig. 5 is an enlarged, left perspective view of a jaw member of the forceps of Fig. 1A;
Fig. 6A is an internal, enlarged, side view of the forceps showing a handswitch having a lockout mechanism in open configuration in according to one aspect of the present disclosure;
Fig. 6B is an internal, enlarged, side view of the locking mechanism of Fig. 6A in locking configuration according to one aspect of the present disclosure;
Figs. 7A-B show schematic top views of the lockout mechanism of Fig. 6A;
Fig. 8 is a schematic diagram of a handswitch having an electrical deactivation switch according to the present disclosure; and
Fig. 9 is a perspective view of an electrosurgical endoscopic forceps according to the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail. Those skilled in the art will understand that the handswitch deactivation mechanisms according to the present disclosure may be adapted for use with either monopolar or bipolar electrosurgical systems and either open or endoscopic instruments.

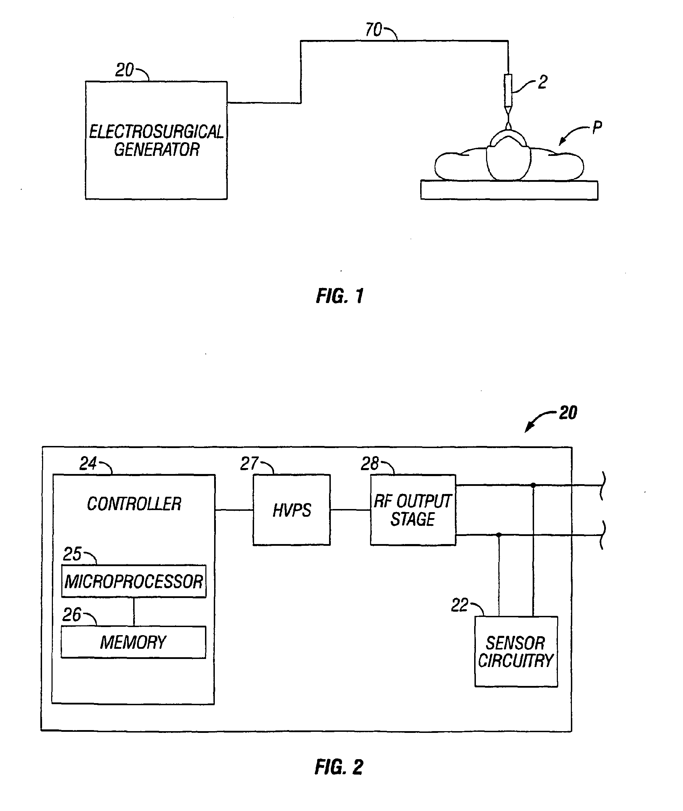

Fig. 1 is a schematic illustration of an electrosurgical system according to one embodiment of the present disclosure. The system includes an electrosurgical instrument 2 having one or more electrodes for treating tissue of a patient P. The instrument 2 may be either of monopolar type including one or more active electrodes (e.g., electrosurgical cutting probe, ablation electrode(s), etc.) or of bipolar type including one or more active and return electrodes (e.g., electrosurgical sealing forceps). Electrosurgical RF energy is supplied to the instrument 2 by a generator 20 via an electrosurgical cable 70, which is connected to an active output terminal, allowing the instrument 2 to coagulate, seal, ablate and/or otherwise treat tissue.

If the instrument 2 is of monopolar type, then energy may be returned to the generator 20 through a return electrode (not explicitly shown), which may be one or more electrode pads disposed on the patient's body. The system may include a plurality of return electrodes that are arranged to minimize the chances of damaged tissue by maximizing the overall contact area with the patient P. In addition, the generator 20 and the monopolar return electrode may be configured for monitoring so-called "tissue-to-patient" contact to insure that sufficient contact exists therebetween to further minimize chances of tissue damage.

If the instrument 2 is of bipolar type, the return electrode is disposed in proximity to the active electrode (e.g., on opposing jaws of bipolar forceps). The generator 20 may also include a plurality of supply and return terminals and a corresponding number of electrode leads.

The generator 20 includes input controls (e.g., buttons, activators, switches, touch screen, etc.) for controlling the generator 20. In addition, the generator 20 may include one or more display screens for providing the user with variety of output information (e.g., intensity settings, treatment complete indicators, etc.). The controls allow the user to adjust power of the RF energy, waveform, and other parameters to achieve the desired waveform suitable for a particular task (e.g., coagulating, tissue sealing; intensity setting, etc.). The instrument 2 may also include a plurality of input controls that may be redundant with certain input controls of the generator 20. Placing the input controls at the instrument 2 allows for easier and faster modification of RF energy parameters during the surgical procedure without requiring interaction with the generator 20.

Fig. 2 shows a schematic block diagram of the generator 20 having a controller 24, a high voltage DC power supply 27 ("HVPS") and an RF output stage 28. The HVPS 27 provides high voltage DC power to an RF output stage 28 which then converts high voltage DC power into RF energy and delivers the RF energy to the active electrode. In particular, the RF output stage 28 generates sinusoidal waveforms of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 generates a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop wherein sensor circuitry 22, which may include a plurality of sensors measuring a variety of tissue and energy properties (e.g., tissue impedance, tissue temperature, output current and/or voltage, etc.), provides feedback to the controller 24. Such sensors are within the purview of those skilled in the art. The controller 24 then signals the HVPS 27 and/or RF output stage 28, which then adjust DC and/or RF power supply, respectively. The controller 24 also receives input signals from the input controls of the generator 20 or the instrument 2. The controller 24 utilizes the input signals to adjust power outputted by the generator 20 and/or performs other control functions thereon.

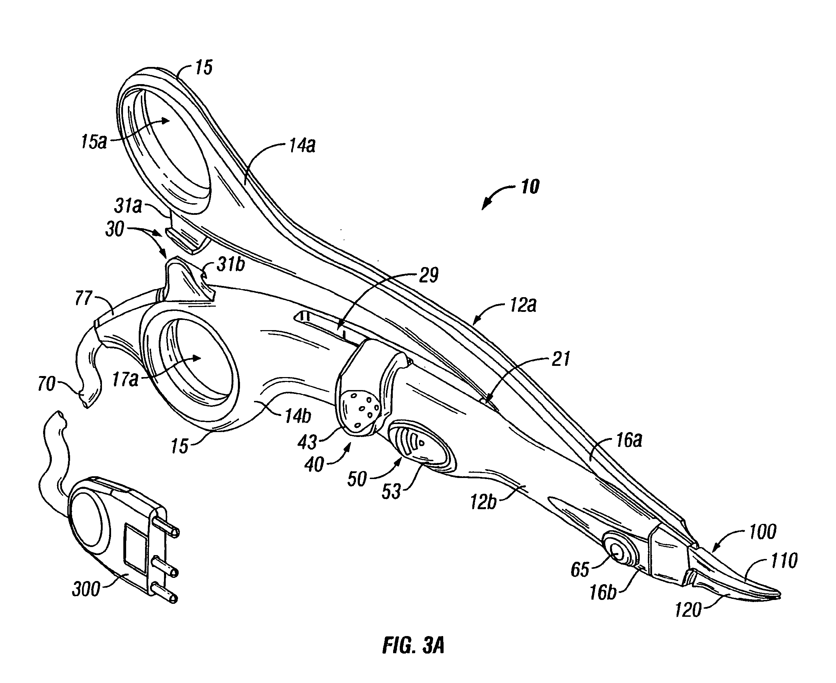

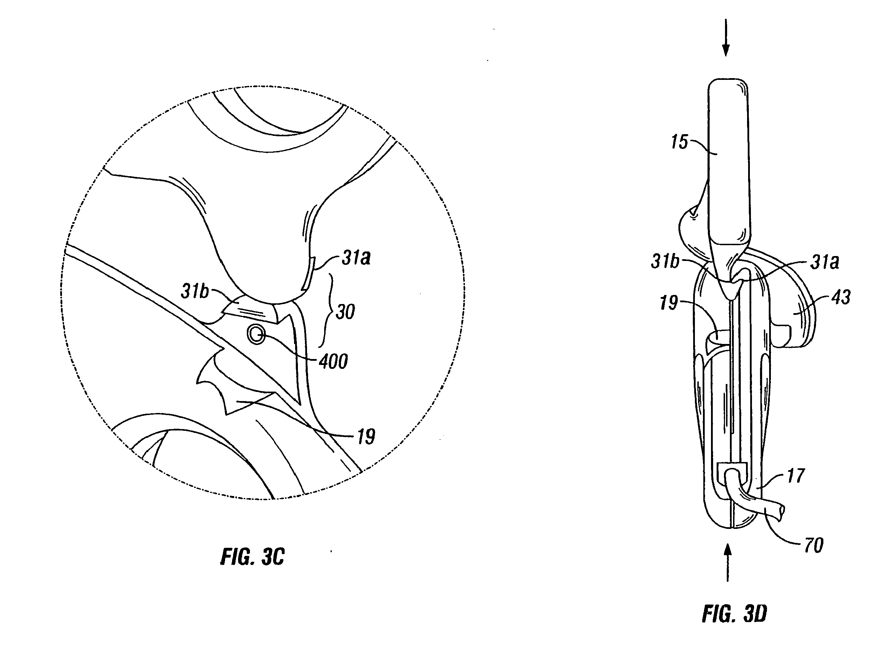

Referring now to Figs. 3A-3E, the instrument 2 is shown as a forceps 10 for use with open surgical procedures. The forceps 10 is connected to the generator 20 via the cable 70 which includes a plug 300 configured for interfacing with an output port (not explicitly shown) of the generator 20.

The forceps 10 includes elongated shaft portions 12a and 12b each having a proximal end 14a, 14b and a distal end 16a and 16b, respectively. In the drawings and in the descriptions which follow, the term "proximal", as is traditional, will refer to the end of the forceps 10 which is closer to the user, while the term "distal" will refer to the end which is further from the user. The forceps 10 includes an end effector assembly 100 which attaches to the distal ends 16a and 16b of shafts 12a and 12b, respectively. As explained in more detail below, the end effector assembly 100 includes pair of opposing jaw members 110 and 120 which are pivotably connected about a pivot pin 65 and which are movable relative to one another to grasp tissue.

Preferably, each shaft 12a and 12b includes a handle 15 and 17, respectively, disposed at the proximal end 14a and 14b thereof which each define a finger hole 15a and 17a, respectively, therethrough for receiving a finger of the user. As can be appreciated, finger holes 15a and 17a facilitate movement of the shafts 12a and 12b relative to one another which, in turn, pivot the jaw members 110 and 120 from an open position wherein the jaw members 110 and 120 are disposed in spaced relation relative to one another to a clamping or closed position wherein the jaw members 110 and 120 cooperate to grasp tissue therebetween.

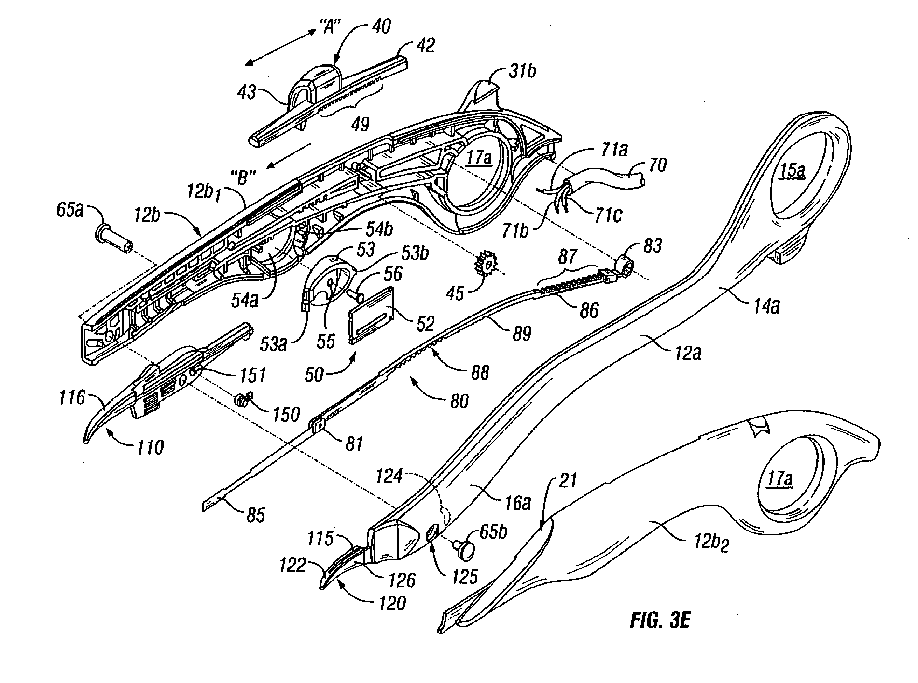

As best seen in Fig. 3E, shaft 12b is constructed from two components, namely, 12b1 and 12b2, which matingly engage one another about the distal end 16a of shaft 12a to form shaft 12b. It is envisioned that the two component halves 12b1 and 12b2 may be ultrasonically-welded together at a plurality of different weld points or the component halves 12b1 and 12b2 may be mechanically engaged in any other known fashion, snap-fit, glued, screwed, etc. After component halves 12b1 and 12b2 are welded together to form shaft 12b, shaft 12a is secured about pivot 65 and positioned within a cut-out or relief 21 defined within shaft portion 12b2 such that shaft 12a is movable relative to shaft 12b. More particularly, when the user moves the shaft 12a relative to shaft 12b to close or open the jaw members 110 and 120, the distal portion of shaft 12a moves within cutout 21 formed within portion 12b2. It is envisioned that configuring the two shafts 12a and 12b in the fashion facilitates gripping and reduces the overall size of the forceps 10 which is especially advantageous during surgeries in small cavities.

As best illustrated in Fig. 3A-3B, one of the shafts, e.g., 12b, includes a proximal shaft connector 77 which is designed to connect the forceps 10 to the generator 20. The proximal shaft connector 77 electromechanically engages the cable 70 such that the user may selectively apply electrosurgical energy as needed. Alternatively, the cable 70 may be feed directly into shaft 12b (or 12a). The cable 70 is coupled to the plug 300 which interfaces with the generator 20.

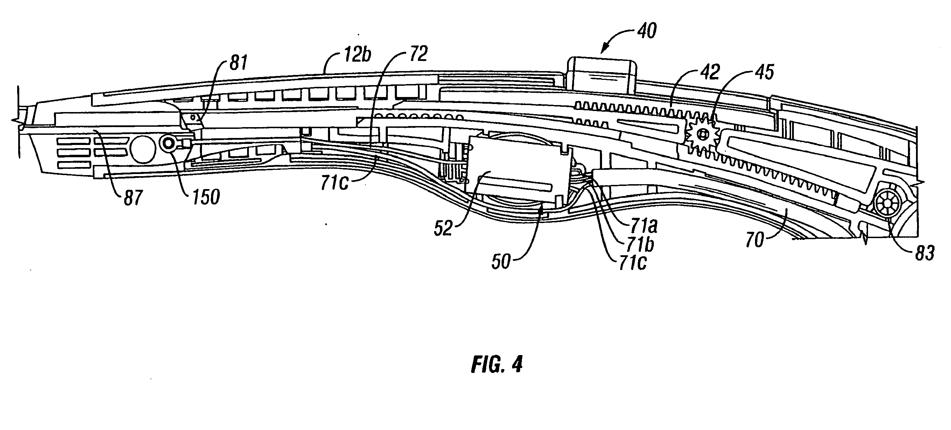

As explained in more detail below, the distal end of the cable 70 connects to a handswitch 50 to permit the user to selectively apply electrosurgical energy as needed to seal tissue grasped between jaw members 110 and 120. More particularly, the interior of cable 70 houses leads 71a, 71b and 71c which upon activation of the handswitch 50 conduct the different electrical potentials from the electrosurgical generator to the jaw members 110 and 120 (See Fig. 4). As can be appreciated, positioning the switch 50 on the forceps 10 gives the user more visual and tactile control over the application of electrosurgical energy. These aspects are explained below with respect to the discussion of the handswitch 50 and the electrical connections associated therewith.

The two opposing jaw members 110 and 120 of the end effector assembly 100 are pivotable about pin 65 from the open position to the closed position for grasping tissue therebetween. The pivot pin connects through aperture 125 in jaw member 120 and aperture 111 disposed through jaw member 110. Pivot pin 65 typically consists of two component halves 65a and 65b which matingly engage and pivotably secure the shafts 12a and 12b during assembly such that the jaw members 110 and 120 are freely pivotable between the open and closed positions. For example, the pivot pin 65 may be configured to be spring loaded such that the pivot snap-fits together at assembly to secure the two shafts 12a and 12b for rotation about the pivot pin 65.

The tissue grasping portions of the jaw members 110 and 120 are generally symmetrical and include similar component features which cooperate to permit facile rotation about pivot pin 65 to effect the grasping and sealing of tissue. As a result and unless otherwise noted, jaw member 110 and the operative features associated therewith are initially described herein in detail and the similar component features with respect to jaw member 120 will be briefly summarized thereafter. Moreover, many of the features of the jaw members 110 and 120 are described in detail in commonly-owned U.S. Patent Application Serial Nos. 10/284,562, 10/116,824, 09/425,696, 09/178,027 and PCT Application Serial No. PCT/US01/11420 the contents of which are all hereby incorporated by reference in their entirety herein.

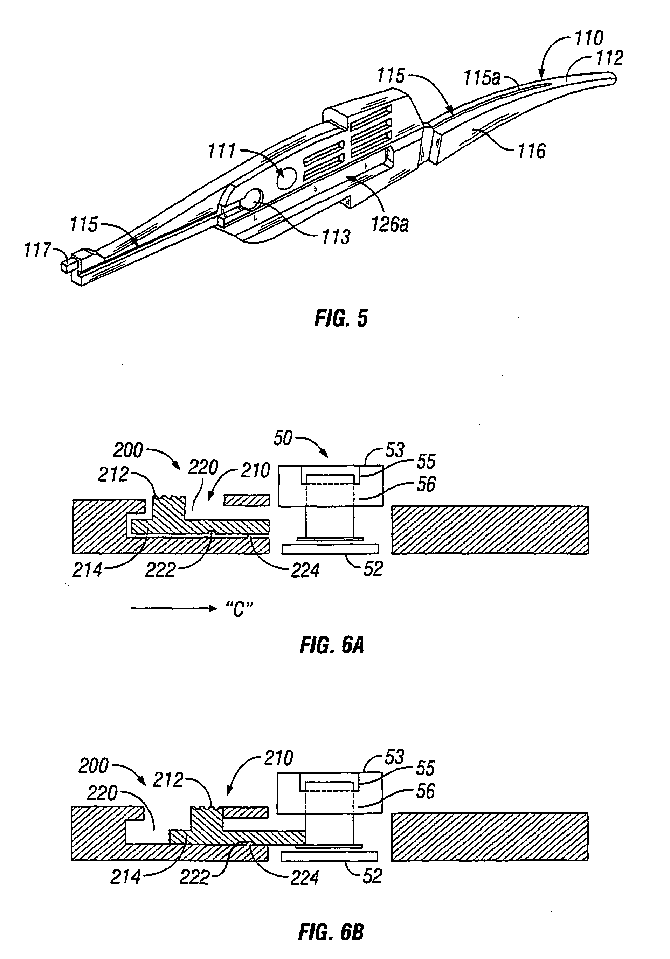

As best shown in Fig. 5, jaw member 110 includes an insulated outer housing 116 which is dimensioned to mechanically engage an electrically conductive sealing surface 112. The outer insulative housing 116 extends along the entire length of jaw member 110 to reduce alternate or stray current paths during sealing and/or incidental damage to tissue. The electrically conductive surface 112 conducts electrosurgical energy of a first potential to the tissue upon activation of the handswitch 50. Insulated outer housing 116 is dimensioned to securely engage the electrically conductive sealing surface 112. It is envisioned that this may be accomplished by stamping, by overmolding, by overmolding a stamped electrically conductive sealing plate and/or by overmolding a metal injection molded seal plate. Other methods of affixing the seal surface 112 to the outer housing 116 are described in detail in one or more of the above-identified references. The jaw members 110 and 120 are typically made from a conductive material and powder coated with an insulative coating to reduce stray current concentrations during sealing.

Likewise, as shown in Fig. 3E, jaw member 120 includes similar elements which include: an outer housing 126 which engages an electrically conductive sealing surface 122 and an electrically conducive sealing surface 122 which conducts electrosurgical energy of a second potential to the tissue upon activation of the handswitch 50.

As best seen in Figs. 5 and 3E, the jaw members 110 and 120 include a knife channel 115 disposed therebetween which is configured to allow reciprocation of a cutting mechanism 80 therewithin. One example of a knife channel is disclosed in WO 02/080797.

It is envisioned that the knife channel 115 may be tapered or some other configuration which facilitates or enhances cutting of the tissue during reciprocation of the cutting mechanism 80 in the distal direction. Moreover, the knife channel 115 may be formed with one or more safety features which prevent the cutting mechanism 80 from advancing through the tissue until the jaw members 110 and 120 are closed about the tissue.

The arrangement of shaft 12b is slightly different from shaft 12a. More particularly, shaft 12b is generally hollow to define a chamber 28 therethrough which is dimensioned to house the handswitch 50 (and the electrical components associated therewith), the actuating mechanism 40 and the cutting mechanism 80. As best seen in Figs. 4 and 3E, the actuating mechanism 40 includes a rack and pinion system having first and second gear tracks 42 and 86, respectively, and a pinion 45 to advance the cutting mechanism 80. More particularly, the actuating mechanism 40 includes a trigger or finger tab 43 which is operatively associated with a first gear rack 42 such that movement of the trigger or finger tab 43 moves the first rack 42 in a corresponding direction. The actuating mechanism 40 mechanically cooperates with a second gear rack 86 which is operatively associated with a drive rod 89 and which advances the entire cutting mechanism 80. Drive rod 89 includes a distal end 81 which is configured to mechanically support the cutting blade 85 and which acts as part of a safety lockout mechanism as explained in more detail below.

Interdisposed between the first and second gear racks 42 and 86, respectively, is a pinion gear 45 which mechanically meshes with both gear racks 42 and 86 and converts proximal motion of the trigger 43 into distal translation of the drive rod 89 and vice versa. More particularly, when the user pulls the trigger 43 in a proximal direction within a predisposed channel 29 in the shaft 12b (See arrow "A" in Fig. 3E), the first rack 42 is translated proximally which, in turn, rotates the pinion gear 45 in a counter-clockwise direction. Rotation of the pinion gear 45 in a counter-clockwise direction forces the second rack 86 to translate the drive rod 89 distally (See arrow "B" in Fig. 3E) which advances the blade 85 of the cutting mechanism 80 through tissue grasped between jaw members 110 and 120, i.e., the cutting mechanism 80, e.g., knife, blade, wire, etc., is advanced through channel 115 upon distal translation of the drive rod 89.

A spring 83 may be employed within chamber 28 to bias the first rack 42 upon proximal movement thereof such that upon release of the trigger 43, the force of the spring 83 automatically returns the first rack 42 to its distal most position within channel 29. The spring 83 may be operatively connected to bias the second rack 86 to achieve the same purpose.

The proximal portion of jaw member 120 also includes a guide slot 124 defined therethrough which allows a terminal connector 150 or so called "POGO" pin to ride therein upon movement of the jaw members 110 and 120 from the open to closed positions. The terminal connector 150 is typically seated within a recess 113 of the jaw member 110. In addition, the proximal end includes an aperture 125 defined therethrough which houses the pivot pin 65. The terminal connector 150 moves freely within slot 124 upon rotation of the jaw members 110 and 120. It is envisioned that the terminal connector 150 is seated within aperture 151 within jaw member 110 and rides within slot 124 of jaw member 120 to provide a "running" or "brush" contact to supply electrosurgical energy to jaw member 120 during the pivoting motion of the forceps 10.

The jaw members 110 and 120 are electrically isolated from one another such that electrosurgical energy can be effectively transferred through the tissue to form a tissue seal. Each jaw member, e.g., 110, includes a uniquely-designed electrosurgical cable path disposed therethrough which transmits electrosurgical energy to the electrically conductive sealing surface 112. It is envisioned that the jaw members 110 and 120 may include one or more cable guides or crimp-like electrical connectors to direct the cable leads towards electrically conductive sealing surfaces 112 and 122. Preferably, cable leads are held securely along the cable path to permit pivoting of the jaw members 110 and 120 about pivot 65.

In operation, the user simply utilizes the two opposing handle members 15 and 17 to grasp tissue between jaw members 110 and 120. The user then activates the handswitch 50 to provide electrosurgical energy to each jaw member 110 and 120 to communicate energy through the tissue held therebetween to effect a tissue seal (See Figs. 21 and 22). Once sealed, the user activates the actuating mechanism 40 to advance the cutting blade 85 through the tissue to sever the tissue along the tissue seal to create a division between tissue halves.

Figs. 3A-3D show a ratchet 30 for selectively locking the jaw members 110 and 120 relative to one another in at least one position during pivoting. A first ratchet interface 31a extends from the proximal end 14a of shaft member 12a towards a second ratchet interface 31 b on the proximal end 14b of shaft 12b in general vertical registration therewith such that the inner facing surfaces of each ratchet 31 a and 31 b abut one another upon closure of the jaw members 110 and 120 about the tissue. It is envisioned that each ratchet interface 31 a and 31 b may include a plurality of step-like flanges (not shown) which project from the inner facing surface of each ratchet interface 3 1 a and 3 1 b such that the ratchet interfaces 3 1 a and 31b interlock in at least one position. Preferably, each position associated with the cooperating ratchet interfaces 31 a and 31 b holds a specific, i.e., constant, strain energy in the shaft members 12a and 12b which, in turn, transmits a specific closing force to the jaw members 110 and 120.

It is envisioned that the ratchet 30 may include graduations or other visual markings which enable the user to easily and quickly ascertain and control the amount of closure force desired between the jaw members. It is envisioned that the shafts 12a and 12b may be manufactured from a particular plastic material which is tuned to apply a particular closure pressure within the above-specified working range to the jaw members 110 and 120 when ratcheted. As can be appreciated, this simplified the manufacturing process and eliminates under pressurizing and over pressurizing the jaw members 110 and 120 during the sealing process.

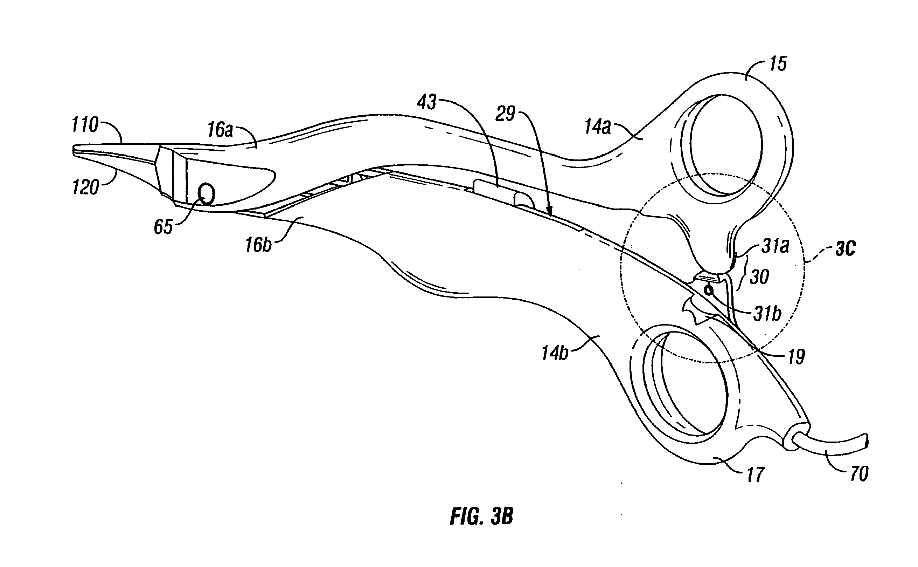

The proximal connector 77 may include a stop or protrusion 19 (See Figs. 3B-D) which prevents the user from over pressurizing the jaw members 110 and 120 by squeezing the handle 15 and 17 beyond the ratchet positions. As can be appreciated this facilitates consistent and effective sealing due to the fact that when ratcheted, the forceps 10 are automatically configured to maintain the necessary closure pressure (about 3 kg/cm² to about 16 kg/cm²) between the opposing jaw members 110 and 120, respectively, to effect sealing. It is known that over pressurizing the jaw members may lead to ineffective tissue sealing.

Fig. 3E and 4 show the electrical details relating to the switch 50. More particularly and as mentioned above, cable 70 includes three electrical leads 71a, 71b and 71c which are fed through shaft 12b. The cable leads 71 a, 71b and 71c are protected by two insulative layers, an outer protective sheath which surrounds all three leads 71a, 71b and 71c and a secondary protective sheath which surrounds each individual cable lead, 71a, 71b and 71c, respectively. The two electrical potentials are isolated from one another by virtue of the insulative sheathing surrounding each cable lead 7 1 a, 7 1 b and 7 1 c. The electrosurgical cable 70 is fed into the bottom of shaft 12b and is held securely therein by one or more mechanical interfaces (not explicitly shown).

Lead 71c extends directly from cable 70 and connects to jaw member 120 to conduct the second electrical potential thereto. Leads 71a and 71b extend from cable 70 and connect to a circuit board 52. The leads 71a-71b are secured to a series of corresponding contacts extending from the circuit board 52 by a crimp-like connector (not explicitly shown) or other electromechanical connections which are commonly known in the art, e.g., IDC connections, soldering, etc. The leads 71a-71b are configured to transmit different electrical potentials or control signals to the circuit board 52 which, in turn, regulates, monitors and controls the electrical energy to the jaw members 110 and 120. More particularly as seen in Fig. 4, the electrical leads 71a and 71b are electrically connected to the circuit board 52 such that when the switch 50 is depressed, a trigger lead 72 carries the first electrical potential from the circuit board 52 to jaw member 110. As mentioned above, the second electrical potential is carried by lead 71c directly from the generator 20 to jaw member 120 through the terminal connector 150 as described above.

As best shown in Figs. 3A and 3E, switch 50 includes an ergonomically dimensioned toggle plate 53 which substantially conforms to the outer shape of housing 20 (once assembled). The toggle plate 53 is positioned in electro-mechanical communication with the circuit board 52 along one side of shaft 12b to facilitate activation of switch 50. As can be appreciated, the position of the switch cap 53 enables the user to easily and selectively energize the jaw members 110 and 120 with a single hand. It is envisioned that the switch cap 53 may be hermetically-sealed to avoid damage to the circuit board 52 during wet operating conditions. In addition, it is contemplated that by positioning the switch cap 53 at a side of the forceps 10 the overall sealing process is greatly simplified and ergonomically advantageous to the user, i.e., after closure, the user's finger is automatically poised for advancement of the cutting mechanism 80.

The toggle plate 53 includes a pair of prongs 53a and 53b extend distally and mate with a corresponding pair of mechanical interfaces 54a and 54b disposed within shaft 12b. Prongs 53a and 53b preferably snap-fit to the shaft 12b during assembly. Toggle plate 53 also includes a switch interface 55 which mates with a switch button 56 which, in turn, connects to the circuit board 52. When the toggle plate 53 is depressed the switch button 56 is pushed against the circuit board 52 thereby actuating the handswitch 50.

Several different types of handswitches 50 are envisioned, for example, switch 50 is a regular push-button style switch but may be configured more like a toggle switch which permits the user to selectively activate the forceps 10 in a variety of different orientations, i.e., multi-oriented activation, which simplifies activation. One particular type of handswitch is disclosed in WO 02/080797.

Fig. 6A shows a lockout mechanism 200 according to the present disclosure. The lockout mechanism 200 is configured to prevent activation of the switch 50. The lockout mechanism 200 prevents the switch 50 from being depressed to actuate the switch button 56. The lockout mechanism 200 includes a lockout switch 210 having an actuating knob 212 extending transversally from a lockout bar 214. The actuating knob 212 is affixed to the lockout bar 214. Alternatively, the lockout bar 214 and the actuating knob 212 may be integrally formed. The actuating knob 212 is dimensioned to protrude from the side of shaft 12b when assembled and may include a variety of protrusions configured to facilitate gripping. The lockout switch 210 may be formed from or coated with an insulative material (e.g., plastics, ceramics) to insulate the lockout switch 210 from any electrical current flowing through the instrument.

The lockout switch 210 is slidably disposed within a guide channel 220 of the shaft 12b such that the lockout switch 210 is selectively moveable in the direction "C" therein. The lockout switch 210 may be disposed facing any direction toward the handswitch 50 and is configured to slide within the shaft 12b. As the actuating knob 212 is moved along the outside of the shaft 12b the lockout bar 214 moves correspondingly therein. In open configuration, the lockout switch 210 is moved away from the switch 50 opposite the direction "C." This allows the toggle plate 53, when depressed, to push the switch button 56 into contact with the circuit board 52 and thereby toggle application of electrosurgical energy. In locking configuration as shown in Fig. 6B, the lockout switch 210 is slid in the direction "C" such that the lockout bar 214 is disposed at least partially between the toggle plate 53 and the circuit board 52. In locking configuration, when the toggle plate 53 is depressed the toggle plate 53 pushes against the lockout bar 214 and is prevented from actuating the switch button 56. The lockout bar 214 may be either in frictional contact with the toggle plate 53 or a predetermined distance away therefrom such that the movement of the toggle plate 53 is still limited.

The lockout mechanism 200 may further include one or more tactile feedback elements, namely a detent 224 disposed within the guide channel 220 and a groove 222 configured to interface with the detent 224. The groove 222 is disposed at the lockout bar 214 on the same longitudinal axis as the detent 224 such that when the lockout switch 210 is moved in the direction "C" the groove 222 interfaces with the detent 224 providing tactile feedback to the user. The groove 222 and the detent 224 are also dimensioned to provide frictional contact between the lockout switch 210 and the shaft 12b and prevent the lockout switch 210 from sliding out of locking configuration.

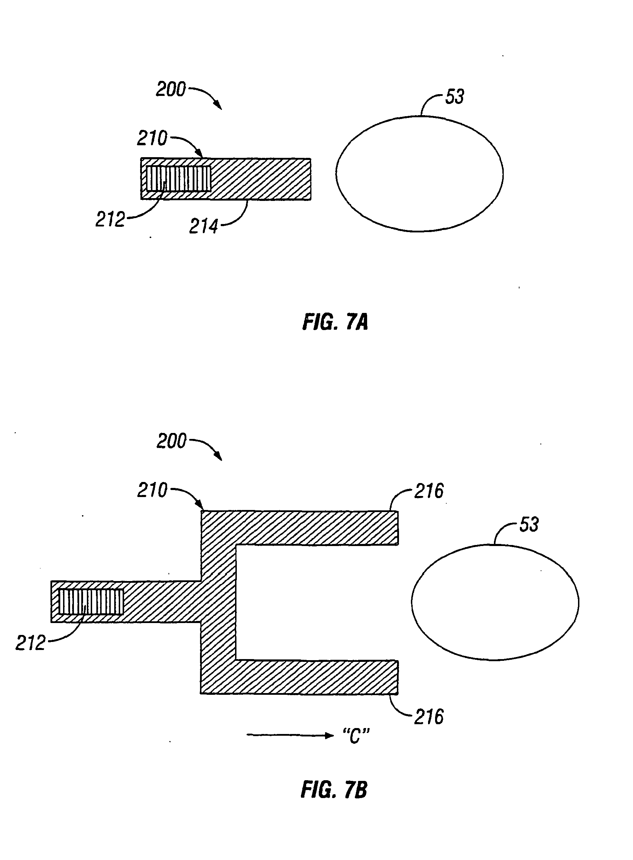

Figs. 7A-B show different embodiments of the lockout mechanism 200. The lockout switch 210 can be formed in a variety of shapes and sizes. As shown in Fig. 7A, the lockout switch 210 may include the lockout bar 214 having an elongated shape. Fig. 7B shows the lockout switch 210 having a so-called U-shaped lock 216 which slides into position below the toggle plate 53. The toggle plate 53 may include a guide channel or a groove (not explicitly shown) disposed therein which are configured to interface with the lockout bar 214 and/or the U-shaped lock 216 when the lockout switch 210 is slid into locking configuration.

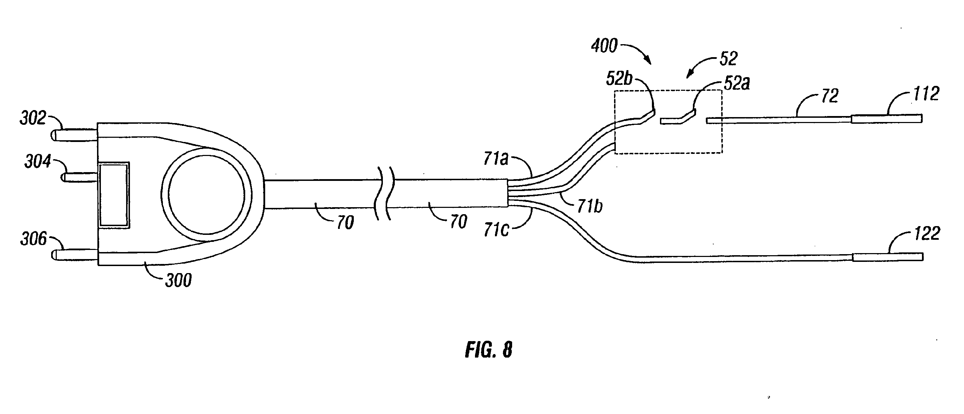

In addition to mechanical lockout mechanisms 200 illustrated in Figs. 6A-B and 7A-B various electrical and electro-mechanical lockout mechanisms are contemplated. Fig. 8 shows an electrical lockout mechanism 400. The plug 300 of the forceps 10 is plugged into the generator 20 and includes a plurality of prongs 302, 304 and 306 connecting to the corresponding leads 71a, 71b and 71c. The prong 306 provides a direct connection for sealing plate 122 to the generator 20 via the lead 71 c. The prongs 302 and 304 are connected to the circuit board 52 via the leads 71a and 71b. The circuit board 52 is connected to the sealing plate 112 via the lead 72. During operation, the switch 50 actuates the switch button 56 which contacts the circuit board 52. The circuit board includes an activation switch 52a which is connected in series with the sealing plate 112 and the generator 20. The switch 52a is toggled via the switch button 56. If the activation switch 52a is closed and tissue is grasped between the sealing plates 112 and 122 then the circuit is complete and electrosurgical energy is transmitted to the tissue. The circuit board 52 also includes a safety switch 52b which is also in series with the actuation switch 52a. As long as either of the switches is open, the circuit is not complete and no electrosurgical energy is supplied to the tissue.

The safety switch 52b may be toggled via a lockout push button disposed anywhere along the forceps 10. The lockout push button may be either manually or automatically actuated. In particular, the automatic actuation of the lockout push button may be accomplished by closure of the forceps 10. As shown in Fig. 3C, the lockout push button 400 may be disposed on inner facing surface of the second ratchet interface 31b such that during closure of the forceps 10 when the first and second interfaces 31a and 31b, respectively, abut one another, the lockout push button 400 is activated (i.e., the schematically-illustrated safety switch 52b is closed) allowing selective application of electrosurgical energy.

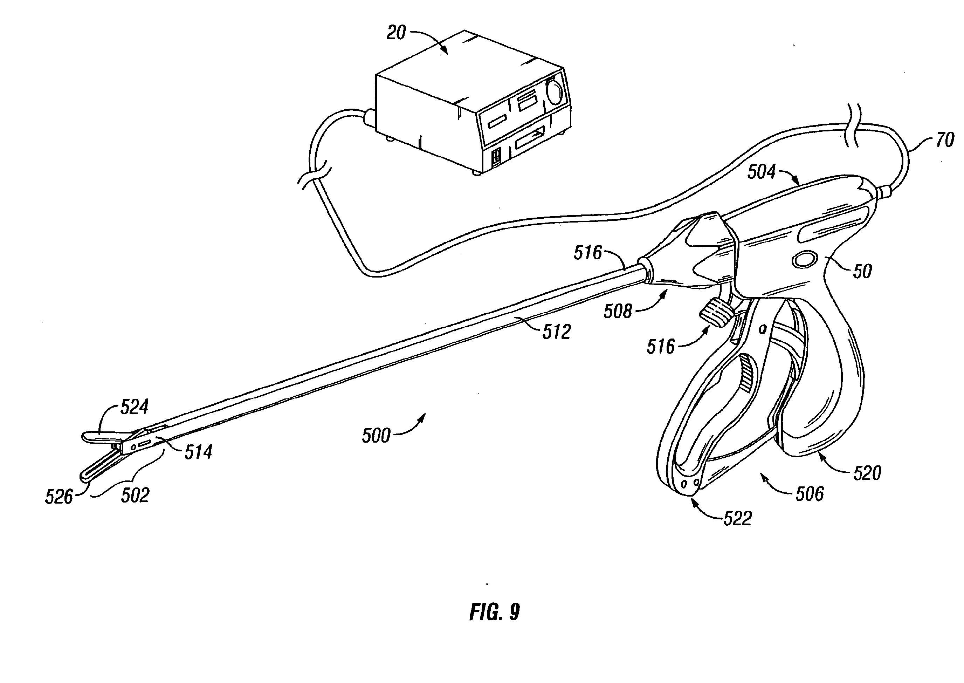

From the foregoing and with reference to the various figure drawings, those skilled in the art will appreciate that certain modifications can also be made to the present disclosure without departing from the scope of the same. For example and as mentioned above, it is contemplated that any of the lockout mechanisms disclosed herein may be employed in an endoscopic forceps such as the endoscopic forceps 500 disclosed in Fig. 9.

Fig. 9 shows the forceps 500 which is configured to support an end effector assembly 502 at a distal end thereof. More particularly, forceps 500 generally includes a housing 504, a handle assembly 506, a rotating assembly 508, and a trigger assembly 510 that mutually cooperate with the end effector assembly 502 to grasp, seal and, if required, divide tissue.

The forceps 500 also includes a shaft 512 that has a distal end 514 which mechanically engages the end effector assembly 502 and a proximal end 516 which mechanically engages the housing 504 proximate the rotating assembly 508. In the drawings and in the description which follows, the term "proximal", refers to the end of the forceps 500 which is closer to the user, while the term "distal" refers to the end of the forceps which is further from the user.

Handle assembly 506 includes a fixed handle 520 and a movable handle 522. Handle 522 moves relative to the fixed handle 520 to actuate the end effector assembly 502 and enables a user to grasp and manipulate tissue.

The end effector assembly 502 includes a pair of opposing jaw members 524 and 526 each having an electrically conductive sealing plate (not explicitly shown), respectively, attached thereto for conducting electrosurgical energy through tissue held therebetween. More particularly, the jaw members 524 and 526 move in response to movement of the handle 522 from an open position to a closed position. In open position the sealing plates are disposed in spaced relation relative to one another. In a clamping or closed position the sealing plates cooperate to grasp tissue and apply electrosurgical energy thereto once the user activates the handswitch 50 which is disposed on the housing 504.

The jaw members 524 and 526 are activated using a drive assembly (not shown) enclosed within the housing 504. The drive assembly cooperates with the movable handle 522 to impart movement of the jaw members 524 and 526 from the open position to the clamping or closed position. Examples of handle assemblies are shown and described in commonly-owned U.S. Application Serial No. 10/389,894 entitled "VESSEL SEALER AND DIVIDER AND METHOD MANUFACTURING SAME" and commonly owned U.S. Application Serial No. 10/460,926 entitled "VESSEL SEALER AND DIVIDER FOR USE WITH SMALL TROCARS AND CANNULAS" which are both hereby incorporated by reference herein in their entirety.

In addition, the handle assembly 506 of this particular disclosure may include a four-bar mechanical linkage, which provides a unique mechanical advantage when sealing tissue between the jaw members 524 and 526. For example, once the desired position for the sealing site is determined and the jaw members 524 and 526 are properly positioned, handle 522 may be compressed fully to lock the electrically conductive sealing plates in a closed position against the tissue. Movable handle 522 of handle assembly 506 is ultimately connected to a drive rod (not explicitly shown) housed within the shaft 512 which, together, mechanically cooperate to impart movement of the jaw members 524 and 526 from an open position wherein the jaw 524 and 526 are disposed in spaced relation relative to one another, to a clamping or closed position wherein the jaw members 524 and 526 cooperate to grasp tissue therebetween.

Further details relating to one particular open forceps are disclosed in commonly-owned U.S. Application Serial No. 10/460,926 filed June 13, 2003 entitled "VESSEL SEALER AND DIVIDER FOR USE WITH SMALL TROCARS AND CANNULAS", the entire content of which being incorporated by reference herein.

From the foregoing and with reference to the various figure drawings, those skilled in the art will appreciate that certain modifications can also be made to the present disclosure without departing from the scope of the same. For example, although the electrical connections are preferably incorporated within one shaft 12b and the forceps 10 is intended for right-handed use, it is contemplated the electrical connections may be incorporated within the other shaft 12a depending upon a particular purpose and/or to facilitate manipulation by a left-handed user. Alternatively, the forceps 10 may operated in an upside down orientation for left-handed users without compromising or restricting any operating characteristics of the forceps 10.

It is also contemplated that the forceps 10 (and/or the electrosurgical generator used in connection with the forceps 10) may include a sensor or feedback mechanism (not explicitly shown) which automatically selects the appropriate amount of electrosurgical energy to effectively seal the particularly-sized tissue grasped between the jaw members 110 and 120. The sensor or feedback mechanism may also measure the impedance across the tissue during sealing and provide an indicator (visual and/or audible) that an effective seal has been created between the jaw members 110 and 120. Commonly-owned U.S. Patent Application Serial No. 10/427,832 discloses several different types of sensory feedback mechanisms and algorithms which may be utilized for this purpose. The contents of this application are hereby incorporated by reference herein.

It is envisioned that a safety switch or circuit (not shown) may be employed such that the switch 50 cannot fire unless the jaw members 110 and 120 are closed and/or unless the jaw members 110 and 120 have tissue 400 held therebetween. In the latter instance, a sensor (not explicitly shown) may be employed to determine if tissue is held therebetween. In addition, other sensor mechanisms may be employed which determine pre-surgical, concurrent surgical (i.e., during surgery) and/or post surgical conditions. The sensor mechanisms may also be utilized with a closed-loop feedback system coupled to the electrosurgical generator to regulate the electrosurgical energy based upon one or more pre-surgical, concurrent surgical or post surgical conditions. Various sensor mechanisms and feedback systems are described in commonly-owned, co-pending U.S. Patent Application Serial No. 10/427,832 the entire contents of which are hereby incorporated by reference herein.

It is also envisioned that the mechanical and electrical lockout mechanisms disclosed herein may be included in a single instrument providing redundant lockout systems.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An electrosurgical forceps for sealing tissue, comprising:
at least one handle having at least one shaft member attached thereto, said at least one shaft member having an end effector attached at a distal end thereof, said end effector including a pair of jaw members being movable from a first position in spaced relation relative to one another to at least one subsequent position wherein the jaw members cooperate to grasp tissue therebetween, each of the jaw members including an electrically conductive sealing plate for communicating electrosurgical energy through tissue held therebetween to effect a tissue seal, the electrically conductive sealing plates adapted to connect to an electrosurgical generator;
a handswitch operatively coupled to at least one of said at least one handle and said at least one shaft member, said handswitch adapted to connect to the electrosurgical generator, said handswitch being selectively actuatable to initiate electrosurgical activation of the forceps; and
a lockout switch operatively coupled to at least one of said at least one handle and said at least one shaft member, the lockout switch being movable from a first configuration wherein said lockout switch allows actuation of said handswitch to a second configuration wherein said lockout switch prevents actuation of said handswitch and activation of said forceps.

2. An electrosurgical forceps according to claim 1, wherein the handswitch is a toggle switch and the lockout switch prevents depression of the toggle switch.

3. An electrosurgical forceps according to claim 2, wherein the toggle switch includes a toggle plate, a circuit board and a switch button disposed therebetween.

4. An electrosurgical forceps according to claim 3, wherein the lockout switch in locking configuration is disposed at least partially between the toggle plate and the switch button preventing depression of the switch button.

5. An electrosurgical forceps according to any one of the preceding claims, wherein the lockout switch includes a lockout bar and an actuating knob extending transversally therefrom, the actuating knob is dimensioned to protrude from the first shaft.

6. An electrosurgical forceps according to claim 5, wherein the lockout bar is a U-shaped lock.

7. An electrosurgical forceps according to any one of the preceding claims, wherein the lockout switch is selectively slideable to prevent activation of the handswitch.

8. An electrosurgical forceps according to any one of the preceding claims, wherein the lockout switch is made from an electrically insulative material.

9. An electrosurgical forceps according to any one of the preceding claims, wherein the lockout switch is configured in electrical communication with said handswitch such that both said lockout switch and said handswitch must be electrically closed to allow activation of said forceps.

10. An electrosurgical forceps according to any one of the preceding claims, wherein the electrosurgical forceps is an open electrosurgical forceps having a pair of first and second handles, each of said handles including shaft members having a jaw member disposed at a distal end thereof and a ratchet interface at a proximal end thereof, said jaw members being selectively movable via operation of said handles from a first position in spaced relation relative to one another to at least one subsequent position wherein the jaw members cooperate to grasp tissue therebetween.

11. An electrosurgical forceps according to claim 10, wherein the lockout switch is operatively coupled to at least one ratchet interface, the lockout switch having a first configuration wherein said ratchets are disposed in spaced, non-operative engagement with one another which prevents actuation of said handswitch and a second configuration wherein said ratchets are operatively engaged with one another which allows actuation of said handswitch and activation of said forceps.
